# EUROPEAN PATENT APPLICATION

(11) **EP 3 996 105 A1**
(43) Date of publication of application: **11.05.2022**
(21) Application number: 20206450.7
(22) Date of filing: 09.11.2020
(51) Int. Cl.: G16H 40/40, G05B 23/02, A61B 6/00

(54) **MULTIPHASE LIFTEIME MONITORING DEVICE**

(71) Applicant: Koninklijke Philips N.V., 5656 AG Eindhoven (NL)
(72) Inventor: VOGTMEIER, Gereon, 5656 AE Eindhoven (NL)
(74) Representative: Philips Intellectual Property & Standards

(57) **Abstract**

The invention concerns a monitoring device for monitoring a medical imaging apparatus, a medical imaging apparatus comprising a monitoring device and a method for evaluating a functionality of a medical imaging apparatus. The monitoring device comprises a sensor for measuring at least one parameter of the medical imaging apparatus. Further, it comprises a data processing unit for receiving data from the sensor and for analysing the received data thereby evaluating based on the analysed data, during at least one life cycle of the medical imaging apparatus, whether a functionality of the medical imaging apparatus is maintained. The sensor is configured for measuring the parameter of the medical imaging apparatus during different life cycles of the medical imaging apparatus. The data processing unit is configured for determining the present life cycle of the medical imaging apparatus and is configured for controlling the at least one sensor based on the determined present life cycle of the medical imaging apparatus.

## Description

### FIELD OF THE INVENTION

The invention relates to the field of monitoring devices for a medical imaging apparatus for evaluating a functionality of the medical imaging apparatus, and more specifically to a medical imaging apparatus, a method for evaluating a functionality of the medical imaging apparatus, and a computer program element for evaluating a functionality of a medical imaging apparatus.

### BACKGROUND OF THE INVENTION

Monitoring basic functionalities of a medical imaging apparatus may be necessary and may be essential for the prediction of lifetime of the medical imaging apparatus. Especially degradation and/or changes in performance of the medical imaging apparatus may be monitored which changes may influence a proper operation of the medical imaging apparatus. The changes may be measured by direct and/or indirect effects on the medical imaging apparatus. Current medical imaging apparatuses may be monitored during a phase of the life cycle but not over the whole life cycle of the medical imaging apparatus. The medical imaging apparatus undergoes different phases during its life cycle, for instance the transport of the medical imaging apparatus, the operation phase in a hospital, and/or a maintenance phase. During each different phases of the life cycle, one and the same component and/or parameter of the medical imaging apparatus may undergo different conditions which influence the functionality of the medical imaging apparatus.

### SUMMARY OF THE INVENTION

Therefore, there exist a need for optimizing a monitoring of a functionality of a medical imaging apparatus. In particular, there exist a need for being able to monitor the functionality of the medical imaging apparatus during the whole life cycle of the medical imaging apparatus, such that not only a partial extract of the lifetime of the medical imaging device is displayed.

An object of the invention is to provide a monitoring apparatus, a medical imaging apparatus comprising the monitoring apparatus and a methodology for evaluating a functionality of the medical imaging apparatus during the whole life cycle of the medical imaging apparatus. Depending on the monitored functionality of the medical imaging apparatus, a prediction of the lifetime of the medical imaging apparatus can be improved.

The object of the present invention is solved with the subject matter of the independent claims, wherein further embodiments are incorporated in the dependent claims.

According to a first aspect of the present invention, a monitoring device for monitoring a medical imaging apparatus comprises at least one sensor configured for measuring at least one parameter of the medical imaging apparatus. The monitoring device further comprises a data processing unit configured for receiving data from the at least one sensor and for analyzing the received data thereby evaluating based on the analyzed data, during at least one life cycle of the medical imaging apparatus, whether a functionality of the medical imaging apparatus is maintained. The sensor is configured for measuring the parameter of the medical imaging apparatus during different life cycles of the medical imaging apparatus. The data processing unit is configured for determining the present life cycle of the medical imaging apparatus and is configured for controlling the at least one sensor based on the determined present life cycle of the medical imaging apparatus.

In the context of the present invention, the term "medical imaging apparatus" shall be understood to describe any kind of medical apparatus suitable to perform imaging of a patient and able to generate a medical image of a patient or body parts of the patient. In particular, the medical imaging apparatus may not be limited to any specific medical imaging process. The described examples and embodiments herein below may be described in reference to an X-ray imaging device, in particular an X-ray tube, wherein this may not be understood limiting the application of the monitoring device. In addition, other medical imaging apparatuses may be monitored by the monitoring device.

In the context of the present invention, the term "parameter of the medical imaging apparatus" shall be understood to describe a measurable physical value, which may influence the functionality, e.g. the operation, of the medical imaging apparatus. The parameter may be any kind of electrical, mechanical, magnetic, optical parameter, which can be measured at the medical imaging apparatus. Further, the measured parameter may not only describe one value it may also be used to describe a range of the value or a specific pattern of the value.

In the context of the present invention, the term "life cycle of the medical imaging apparatus" shall be understood to describe the life time of the medical imaging apparatus which may be divided into different life cycles, for instance different phases, from the beginning, e.g. the mounting of the medical imaging apparatus, until an proper operation of the medical imaging apparatus could not be guaranteed anymore, e.g. the end of the life cycle. This will be explained in more detail hereinafter in the context of the particular embodiments.

In the context of the present invention, the term "present life cycle of the medical imaging apparatus" shall be understood to describe the life cycle in which the medical imaging apparatus currently is when the parameter is measured. Accordingly, the present life cycle is the life cycle in which the monitoring device currently is used and the functionality is determined.

In the context of the present invention, the term "a functionality of the medical imaging apparatus" shall be understood to describe the ability of the medical imaging apparatus to work properly. In particular, the functionality may be understood to describe whether the medical imaging apparatus is able to perform, to still perform, the medical imaging in a good quality. Further, the functionality of the medical imaging apparatus may be the functionality of the whole medical imaging apparatus or from parts of the medical imaging apparatus, for instance from components used in the medical imaging apparatus. The functionality to be maintained may be for instance a manufacturing quality of the medical imaging apparatus, which means the function, which the medical imaging apparatus is able to perform directly after the manufacturing, which may be derived from respective test protocols during the manufacturing. For instance, an X-ray tube may be manufactured, or may be mounted into an medical imaging apparatus, wherein the emitted electron beam, the vacuum and anode parameters are tested during the manufacturing and the functionality of the medical imaging apparatus may be the maintaining of these values of the parameter (for instance in a respective value boundaries allowing deviations which would not influence the functionality). The position of the sensor of the monitoring device at the medical imaging apparatus may be chosen depending on the parameter to be measured. Hence, each parameter, which should be measured, may require another location of the sensor. For instance, the sensor may be placed at the housing of the medical imaging apparatus for measuring a temperature and/or a humidity of the surrounding. Contrary, the sensor may be placed inside the medical imaging apparatus, for example on a printed circuit board for monitoring the temperature thereof. The location of the sensor may not limit the amount of parameters to be measured, hence one location may be suitable to allow a measurement of different parameters.

In other words, the monitoring device presented herein may be used to monitor the medical imaging apparatus directly, which means, wherein the monitoring device may be configured to receive respective signals from the medical imaging apparatus. On the other hand, the monitoring device may be used to monitor a specific component and/or specific components of the medical imaging apparatus, such that at least a part of the medical imaging apparatus is monitored. Therefore, it may not be necessary to monitor all components, e.g. the whole medical imaging apparatus, but only parts of it. By monitoring the medical imaging apparatus or components thereof, changes in performance and degradation of the medical imaging apparatus or the component can be measured and determined. Further, a lifetime of the medical imaging apparatus and/or its component can be predicted and additionally a prediction of maintenance may be made, such that the medical imaging apparatus or components thereof may be repaired or changed before an end of the lifetime.

According to an exemplary embodiment of the invention, the evaluation of the functionality of the medical imaging apparatus may comprise evaluating whether an existing functionality of the medical imaging apparatus is maintained and conforming whether medical imaging apparatus is continuously capable for applying the functionality. Specifically, it may be determined whether the functionality is maintained in specific acceptable boundaries. For instance, whether the surrounding temperature, the temperature of the medical imaging apparatus or a component thereof, i.e. the respective measured parameter(s) is in a range wherein the medical imaging apparatus can still operate properly. Further, it may be determined how the functionality of the medical imaging apparatus may be affected by environmental effects, or user effects, and/or device wearing, wherein this is determined based on the measured parameter(s). The confirmation of the capability of the functionality may be send to the data processing unit and/or further to an indication unit for displaying this functionality to user. Further, the data processing unit may be able to compare previously determined functionalities with the present determined functionality for evaluating whether the functionality is maintained. For instance, the present determined functionality may be equal to a previously determined functionality and/or may be in range of allowed deviations. For example, the functionality may be influenced by at least one of the following: aging and mechanical wear effects which might lead to higher power consumption of the motor; changes in electrical parameters which might require recalibration and/or will lead to a malfunction, e.g. the focusing of the electron beam; changes in the vacuum condition which might lead to arcing events, shock might lead to mechanical damage.

According to an exemplary embodiment of the invention, the different life cycles of the medical imaging apparatus are at least one of a phase of mounting the medical imaging apparatus, a phase before transporting the medical imaging apparatus, a phase of transporting and storing the medical imaging apparatus, a phase of operating the medical imaging apparatus, and a phase of maintenance of the medical imaging apparatus. For each phases the data processing unit is configured for controlling the at least one sensor based on the parameter to be measured in the respective life cycle.

In particular, the phase of mounting the medical imaging apparatus may start already before the first components of the medical imaging apparatus have been mounted. For instance, the medical imaging apparatus may include during this phase a database of all individual components and subcomponents with exact component numbers and geometric information of the mechanical parts. This database may be stored in the monitoring device in a storage unit, or may be stored on an external storage unit used for the monitoring device database. The database may be stored only during production of the medical imaging apparatus but may also be available later and/or transferred to a local product history record. During the phase of mounting the medical imaging apparatus all parts to be mounted may have dedicated identification numbers, which may be checked and synchronized with the database. The monitoring device may be configured to monitor step by step all mounting, manufacturing, processes of the medical imaging apparatus. Alignment data, measurement data and test data received and determined during the mounting of the apparatus may be stored in the database, for instance in a mobile storage unit. As soon as measurements with the sensor of the monitoring device makes sense also these measurements may be logged in the database, wherein too high values of the measured parameter may be indicated as risk and/or quality influencing value. Further, calibration data, e.g. for the bearing balancing of the medical imaging device, may be stored as input for a bearing model as well as vibration measurements as part of the final quality checks. This list may not be limiting, as also further relevant data may be stored.

The phase before transporting the medical imaging apparatus may start after the final product check during the phase of mounting the medical imaging apparatus and may start and also end before the apparatus is transported to a stock or to a costumer. In this phase, all information, e.g. mounting data, which should not stay on the medical imaging apparatus, in particular not in the database, may be transferred and the monitoring functionality with data acquisition parameters for the sensor date may re-configured (may be done automatically). This may mainly include the data logging of temperature, humidity and mechanicals shocks as measured parameter(s) by the monitoring device during storage and transportation to identify risks or damaging events. Further, this phase may comprise a system integration. A typically identifier for this phase may the absence of a power signal, e.g. power supply voltage and/or current as the parameter to be measured. During this phase, when no power supply may be actively connected to the medical imaging apparatus, the data processing unit (and/or the data acquisition unit) may continue data acquisition in a battery powered mode but the measured component or power supply is disconnected.

In the phase of transporting and storing the medical imaging apparatus, also relevant parameters may be logged in the database, which means that they are measured by the monitoring device. In case of critical events, for instance a critical acceleration sensor signal, indicating a mechanical shock of the medical imaging apparatus may cause sending an alarm. In particular, during the phase of transporting and storing the medical imaging apparatus may be transported to a customer, wherein the medical imaging apparatus may be transported alone or when integrated in a medical system.

The phase of operating the medical imaging apparatus may be the phase wherein the medical imaging apparatus is in operation modus at the costumer, e.g. a hospital. For the medical imaging apparatus, e.g. in a CT scanner or a C-arc system all relevant data may be measured and stored including abnormal conditions and degradation effects. For instance, in this phase for a medical imaging apparatus, like an X-ray tube in a CT-gantry, additionally to rotation forces the g-forces may be measured for determining the functionality.

The phase of maintenance of the medical imaging apparatus may be a phase during failure analysis after the replacement by a service team after, for example, a predictive maintenance warning. For instance, a high-resolution measurement of the parameter(s) may help to identify all components of the medical imaging apparatus, which have to be repaired or replaced. After a final performance check, which may be similar to the check at the end of the phase of mounting the medical imaging apparatus, can enable a second life of the refurbished medical imaging apparatus. After the final performance check, the settings for monitoring device may be set equally to the settings of the phase of before transporting.

The boundaries of the phases may not be strictly, it may be possible that the phases comprise further sub phases or that phases may overlap. All phases of the life cycle may have special configurations for the measurement of the parameter and in particular, different parameters may be relevant for the respective phase. For instance, these configurations may relate to data acquisition, trigger level for measurements, sensitivity settings for sensors, time stamps for measurements, alarm level, communication procedures and interface protocols, which will be described in the further embodiments of the invention. Depending on the respective phase of the life cycle the monitoring device choses the sensor and/or the parameter(s) to be measured and the settings for the data acquisition, storage, data processing features etc., wherein the not used settings may not be activated and be transferred in an energy saving mode.

The above described different life cycles of the medical imaging apparatus may also be applicable to a life cycle, e.g. different life cycles, of at least one component used in a medical imaging apparatus and which component should be monitored by the monitoring device.

According to an exemplary embodiment of the invention, a method for determining the functionality of a medical imaging apparatus may comprise the determination of the above mentioned different life cycles and controlling of the at least one sensor based on the parameter to be measured in the respective life cycle.

According to an exemplary embodiment of the invention, the measured parameter is at least one of a vibration of a component of the medical imaging apparatus, a vibration of the medical imaging apparatus, an acceleration of a component of the medical imaging apparatus, an acceleration of the medical imaging apparatus, a surrounding temperature of a component of the medical imaging apparatus, a surrounding temperature of the medical imaging apparatus, a surrounding humidity of a component of the medical imaging apparatus, a surrounding humidity of the medical imaging apparatus, a temperature of the medical imaging apparatus, a temperature of a component of the medical imaging apparatus, a mechanical shock of the medical imaging apparatus, a mechanical shock of a component of the medical imaging apparatus, a vacuum parameter of the medical imaging apparatus, an emitter parameter of the medical imaging apparatus, a power supply parameter of the medical imaging apparatus, an over/under voltage parameter of a component of the medical imaging apparatus, an over/under voltage parameter of the medical imaging apparatus, an over/under current parameter of a component of the medical imaging apparatus, an over/under current parameter of the medical imaging apparatus, an X-ray radiation parameter of the medical imaging apparatus, a rotation speed of a component of the medical imaging of the apparatus relative to the medical imaging apparatus, a rotation speed of the medical imaging apparatus, an electron beam parameter of the medical imaging apparatus, and a magnetic field parameter of the medical imaging apparatus. This listing may not be limiting, other parameters may also be measured. For instance, at least all relevant physical parameters of at least a component of the medical imaging apparatus and/or of the medical imaging apparatus itself may be measured. The sensor may be configured for measuring, an instability parameter of the medical imaging apparatus, and/or disturbing signals disturbing the measurement of the sensor. Disturbing signals may be any signals from surrounding electronically devices, which may influence the measured parameter signal, wherein when measuring the disturbing signals these may be filtered and thereby excluded from the interesting signal of the medical imaging apparatus.

The vibration of the component and/or the medical imaging apparatus may be measured using acceleration sensors, for instance the vibration of the bearing in a rotating system may be measured. The vibration may be any kind of mechanical vibration caused by the medical imaging apparatus itself or by the environment of the medical imaging apparatus.

The acceleration of the component and/or the medical imaging apparatus may be also measured by acceleration sensors. For instance, the acceleration may be the chosen parameter to be measured during the phase of transport and storage, wherein the acceleration may be an acceleration with respect to the ground above which the medical imaging apparatus may be positioned or transported, or it may be an acceleration with respect to a medical imaging system in which the medical imaging apparatus is integrated, and/or in which the component is integrated. For instance, an acceleration may be measured when the X-ray tube may fall to ground during transport, or when it is operated in a rotating CT-gantry.

The surrounding temperature of the component and/or the medical imaging apparatus may be the temperature of the direct surrounding in which the medical imaging apparatus is located.

The surrounding humidity of the component and/or the medical imaging apparatus may be the humidity of the direct surrounding in which the medical imaging apparatus is located.

The temperature of the component and/or the medical imaging apparatus may be preferably measured during the phase of operation. The temperature may be a temperature of the housing which should not exceed a surrounding temperature at normal conditions, for instance between 15°C to 40 °C. Further, it may be a temperature of cooling for the medical imaging apparatus, of a motor oil used in a motor of the medical imaging apparatus or used in any pneumatic parts of the medical imaging apparatus, a cooling oil for an X-ray tube, wherein the cooling oil is the cooling liquid. Moreover, this parameter may be transport or storage temperatures which should be detected and which may indicate an influence of the functionality, for the respective phases, may be even down - 20 or - 40°C or up to + 50°C or more (which may occur when the apparatus is transported, stored in an airplane in desert, or shipment in a container).

The mechanical shock of the component and/or the medical imaging apparatus may be any kind of sudden acceleration caused for example by impact, drop, kick or the like. Mechanical shock describes matter subject to extreme rates of force with respect to time. A shock pulse can be characterized by its peak acceleration, the duration, and the shape of the shock pulse (half sine, triangular, trapezoidal, etc.), wherein a shock response spectrum may be a method for further evaluating a mechanical shock.

The vacuum parameter of the component and/or the medical imaging apparatus may be preferably measured during the phase of operation, wherein for instance a bad vacuum quality may lead to arcing events.

The emitter parameter of the component and/or the medical imaging apparatus may be preferably measured during the phase of operation. The emitter parameter may be an emitter voltage, an emitter current used for generating an X-ray beam, an electron beam or the like.

The over/under voltage parameter of the component and/or the medical imaging apparatus may be preferably measured during the phase of operation, wherein this parameter may be a high voltage parameter of a high voltage unit of an X-ray tube. Any variation of high voltage may influence stress of the X-ray tube. The over/under voltage parameter may be measured for indicating any electrical short cuts influencing the medical imaging apparatus, for example arcing events may be detected or even prevented, which may influence the vacuum quality.

The over/under current parameter of the component and/or the medical imaging apparatus may be preferably measured during the phase of operation.

The X-ray radiation parameter of the component and/or the medical imaging apparatus may be any change in the X-ray radiation parameter. For instance, this parameter may be an intensity of the X-ray radiation and a variation of the intensity of the X-ray radiation. Further, it may be a radiation spectrum of the component and/or the medical imaging apparatus may be a change in the radiation spectra, and spectral change of energy spectrum of X-ray radiation. A change in the spectrum may indicate a change in the operation condition and can give early indications in potential failure modes.

The rotation speed of the component and/or the medical imaging apparatus may be preferably measured during the phase of operation, wherein the rotation speed may be the rotation speed of the component with respect to the medical imaging apparatus or the rotation speed of the medical imaging apparatus with respect to a medical imaging system. The electron beam parameter of the component and/or the medical imaging apparatus may be preferably measured during the phase of operation, wherein this parameter may be the focus of the electron beam.

The magnetic field parameter of the component and/or the medical imaging apparatus may be preferably measured during the phase of operation, wherein the magnetic field parameter may be any kind of magnetic field parameter of an MRI apparatus as the medical imaging apparatus.

Moreover, the sensor may be configured for measuring at least one digital signal of the data processing unit by at least one sensor thereby determining a status of the data processing of the data processing unit. For instance, the measurement of a digital signal may refer to a measurement of a control signal used for controlling the sensor and /or the data acquisition, hence the data processing unit. For preventing failures of data transmission and data processing, these digital signals may be measured and monitored, such that a replacement and/or repair of the component generating a digital signal can be predicted.

According to an exemplary embodiment of the invention, the different life cycles of the medical imaging apparatus are determined depending on the measured parameter of the at least one sensor and/or determined autonomously by the monitoring device and/or are determined based on an external trigger. The external trigger may comprise at least one of a manual activation, and an external sensor signal received by the data processing unit. In particular, the life cycles may be determined based on the presence or absence of a measured signal, or measured parameter. For instance, whether power is disconnected from the medical imaging apparatus or whether no control signal is received at the medical imaging apparatus. Further, the life cycles may be determined based on a detection of typical signal patterns from the sensor, e.g. high g-force either due to transport or due to rotation in a CT gantry. A well-defined signal pattern may be the rotation of any component of the medical imaging apparatus in a CT gantry, which may define a phase of a life cycle. Furthermore, the determination of the respective life cycle may be performed autonomously by the monitoring device itself, which consequently results in an adaption of the sensor configuration for the respective life cycle and a conditioning including storage and processing optimization per phase of the life cycle. The autonomous detection of the different life cycles may be determined by identifying typical input data of the sensor. For instance, for determining the phase of mounting the components of the medical imaging apparatus would not undergo any active mechanical or electrical activity, changes, such that the absence of a power supply signal (voltage, current) and the absence of a motor voltage, motor rotation may indicate this phase. Similarly, the transport and storage phase may comprise the same absence parameter as in the phase of mounting. For both phase therefore the relevant measured parameter may be the temperature, humidity and mechanical shock. The phase of operation of the medical imaging apparatus may be determined based on the presence of a power supply signal, for instance a measured voltage parameter in a respective range of a power supply (e.g. 230 V). Further, relevant parameter indicating the phase of operation may be typically operation cycles of the medical imaging apparatus, such as day and night cycle of operation or breaks during the operation. For instance, the before transport phase may be determined based on a first power supply signal measured by the sensor and/ or a calibration of the sensor. Further, the phase of maintenance may be determined based on a detected failure of the functionality and the need for changing the failed component.

According to an exemplary embodiment of the invention, a method for determining the functionality of a medical imaging apparatus may comprise the determination depending on the measured parameter of the at least one sensor the different life cycles autonomously by the monitoring device itself and/or based on an external trigger as described in the embodiments relating to the monitoring device.

According to an exemplary embodiment of the invention, the data processing unit is configured for controlling a signal condition of the sensor and/or an analysis of the measured parameter based on the determined life cycle of the medical imaging apparatus. The signal condition may be understood as a data acquisition rate of the sensor, an amplification of the sensor signal, and/or a filtration of the sensor signal. In general, the signal condition matches the requirement of the respective life cycle for detecting the condition, i.e. the functionality of the medical imaging apparatus, abnormal and/or normal conditions of the medical imaging apparatus. The data processing unit may be configured to control at least one signal condition (at least one of a data acquisition rate of the sensor, an amplification of the sensor signal, and/or a filtration of the sensor signal) or it may be control a plurality of them.

According to an exemplary embodiment of the invention, a method for determining the functionality of a medical imaging apparatus may comprise the step of controlling a signal condition of the sensor and/or an analysis of the measured parameter based on the determined life cycle of the medical imaging apparatus.

According to an exemplary embodiment of the invention, the at least one sensor is configured for measuring a plurality of parameters of the medical imaging apparatus, wherein the at least one of the plurality of parameters is preferably at least one of a vibration of a component of the medical imaging apparatus, a vibration of the medical imaging apparatus, an acceleration of a component of the medical imaging apparatus, an acceleration of the medical imaging apparatus, a surrounding temperature of a component of the medical imaging apparatus, a surrounding temperature of the medical imaging apparatus, a surrounding humidity of a component of the medical imaging apparatus, a surrounding humidity of the medical imaging apparatus, a temperature of the medical imaging apparatus, a temperature of a component of the medical imaging apparatus, a mechanical shock of the medical imaging apparatus, a mechanical shock of a component of the medical imaging apparatus, a vacuum parameter of the medical imaging apparatus, an emitter parameter of the medical imaging apparatus, a power supply parameter of the medical imaging apparatus, an over/under voltage parameter of a component of the medical imaging apparatus, an over/under voltage parameter of the medical imaging apparatus, an X-ray radiation parameter of the medical imaging apparatus, a rotation speed of a component of the medical imaging of the apparatus relative to the medical imaging apparatus, a rotation speed of the medical imaging apparatus, an electron beam parameter of the medical imaging apparatus, and a magnetic field parameter of the medical imaging apparatus. This listing may not be limiting, other parameter may also be measured. For instance, the sensor may be configured for measuring an instability parameter of the medical imaging apparatus, and/or disturbing signals disturbing the measurement of the sensor. In particular, the sensor may be configured to measure a plurality of parameters, wherein the measured parameters may be a plurality of the same parameter or a plurality of different parameter. For instance, the plurality of parameters may be any one of the parameter described with the embodiment of the single parameter measurement. Moreover, the sensor may be configured for measuring at least one digital signal of the data processing unit by at least one sensor thereby determining a status of the data processing of the data processing unit. For instance, the measurement of a digital signal may refer to a measurement of a control signal used for controlling the sensor and /or the data acquisition, hence the data processing unit. For preventing failures of data transmission and data processing, this digital signal may be measured and monitored, such that a replacement and/or repair of the component generating a digital signal can be predicted.

According to an exemplary embodiment of the invention, the monitoring device further comprises a plurality of sensors configured for measuring at least one parameter and/or a plurality of parameters of the medical imaging apparatus. The monitoring device is configured for evaluating a plurality of functionalities of the medical imaging apparatus during the different life cycles of the medical imaging apparatus. For example, the plurality of functionalities may be based on one measured parameter or the evaluated functionality may be based on a plurality of parameters during the different life cycles.

According to an exemplary embodiment of the invention, the monitoring device may comprise a self-sustainable power supply and/or wherein the monitoring device is configured for being connected to a power supply of the medical imaging apparatus. Specifically, the self-sustainable power supply may be a battery or an accumulator connected to the medical imaging apparatus or connected to a component thereof. The monitoring device may be supplied with power by the power supply system of the medical imaging apparatus or by the self-sustainable power supply or both. The self-sustainable power supply may be an integral part of the monitoring device or may be an exchangeable external part.

According to an exemplary embodiment of the invention, the monitoring may further comprise an indicating unit configured for indicating an optical and/or acoustical signal when the measured parameter of the medical imaging apparatus exceeds a predetermined signal signature, wherein the predetermined signal signature depends on the phase of the life cycle. The predetermined signal signature may be at least one of a predetermined threshold of a value of the measured parameter, a predetermined course of the value of the measured parameter, a predetermined signal form of the (value of the) parameter, a predetermined signal pattern, and a predetermined parameter pattern.

According to an exemplary embodiment of the invention, the data processing unit is configured for storing the data received from the at least one sensor and/or is configured for processing the stored data for at least one of the different life cycles or preferably for all life cycles of the medical imaging apparatus. The monitoring device may comprise a storage unit configured for storing the data received from the sensor, wherein the storage unit may be part of the data processing unit or may be an extra part. Further, the data may be stored on an external unit, like any processing unit capable for storing data, for further processing. The data may be stored or may not be stored, further also processed data may be stored or may not be stored, wherein it can be decided whether the data should be available for later examination, data comparison, calibration of the sensor, calibration of the medical imaging apparatus, and/or failure analysis.

According to a further aspect of the invention, a component, for a medical imaging apparatus, comprises a monitoring device according to any of the above-described embodiments. The monitoring device is arranged at the component, and/or wherein the monitoring device is removably arranged at the component. For instance, the component may be a substantial functional device of the medical imaging apparatus. Further, the component is preferably an X-ray tube. According to this embodiment, the monitoring device may be used for monitoring at least parts of the medical imaging apparatus, i.e. the component. In particular, the component may be monitored itself or may be monitored as a part of the medical imaging apparatus. The component may be monitored during the different life cycles of the medical imaging apparatus, such that the residual lifetime and/or a maintenance prediction of the component could be carried out. On the other hand, the different life cycles of the component may be monitored, wherein the different life cycles of the component may be and/or include a phase, for instance the transport phase, or manufacturing phase, wherein the component is not a part of the medical imaging apparatus and later on the life cycle of the component comprises life cycles, e.g. phases, wherein the component is a functional part of the medical imaging apparatus. The described embodiments referring only to a medical imaging apparatus may not only be limited the medical imaging apparatus, they may also be applicable for a component of the medical imaging apparatus.

According to a further aspect of the invention, a medical imaging apparatus comprises a medical imaging unit for generating a medical imaging of a patient, and a monitoring device according to any of the above-described embodiments. The medical imaging apparatus is preferably a medical X-ray imaging apparatus and/or wherein the monitoring device is arranged at the medical imaging apparatus, and/or wherein the monitoring device is removably arranged at the medical imaging apparatus. On the other hand, the medical imaging apparatus may also be a MRT, PET, SPECT, US or other medical imaging devices may be monitored.

According to a further aspect of the present invention a method for evaluating functionality of a medical imaging apparatus comprises the steps of measuring at least one parameter of the medical imaging apparatus by at least one sensor of a monitoring device and receiving data from the at least one sensor by a data processing unit of the monitoring device. The method may further comprise the step of analyzing the received data by the data processing unit of the monitoring device and thereby evaluating based on the analyzed data, during at least one life cycle of the medical imaging apparatus, whether a functionality of the medical imaging apparatus is maintained. Also the step of measuring the parameter of the medical imaging apparatus by the sensor during different life cycles of the medical imaging apparatus is comprised, as well as the steps of determining the present life cycle of the medical imaging apparatus by the data processing unit, and controlling the at least one sensor, by the data processing unit, based on the determined present life cycle of the medical imaging apparatus.

According to an exemplary embodiment of the invention, the method may further comprise the steps of evaluating whether an existing functionality of the medical imaging apparatus is maintained and conforming whether medical imaging apparatus is continuously capable for applying the functionality and/or the step of controlling, by the data processing unit, a data acquisition rate of the sensor and/or an analysis, by the data processing unit, of the measured parameter based on the determined life cycle of the medical imaging apparatus. Further, the method may comprise the step of measuring a plurality of parameters of the medical imaging apparatus by the at least one sensor and/or the step of measuring at least one parameter and/or a plurality of parameters of the medical imaging apparatus by a plurality of sensors and/or the step of evaluating a plurality of functionalities of the medical imaging apparatus during the different life cycles of the medical imaging apparatus. The different life cycles of the medical imaging apparatus are at least one of a phase of mounting the medical imaging apparatus, a phase before transporting the medical imaging apparatus, a phase of transporting and storing the medical imaging apparatus, a phase of operating the medical imaging apparatus, and a phase of maintenance of the medical imaging apparatus. The method may further comprise the step of controlling for each phases the at least one sensor based on the parameter to be measured in the respective life cycle by the data processing unit of the monitoring device.

According to a further aspect of the invention, the step of analysing may comprise applying a machine learning algorithm and/or artificial intelligence methods for identification of the life cycle. Both the machine learning algorithm and the AI methods may be applied to the measured parameter(s), the determined sensor conditions, and/or the determined functionality for comparison with a previously determined functionality. Hence, these algorithm and methods may be used for lifetime prediction and maintenance prediction of the medical imaging apparatus or a component thereof. For a precise prediction of the maintenance it is important to get ground truth data and other relevant information about the key components that are under inspection, therefore the monitoring device is used.

According to a further aspect of the invention, a computer program element for evaluating a functionality of a medical imaging apparatus is presented, wherein the computer program element, when being executed by a processor of a monitoring device as presented herein, is adapted to cause the monitoring device to measure at least one parameter of the medical imaging apparatus by at least one sensor. Further to receive data from the at least one sensor and for analyzing the received data, by a data processing unit, thereby evaluating based on the analyzed data, during at least one life cycle of the medical imaging apparatus, whether a functionality of the medical imaging apparatus is maintained. The computer program element, when being executed by a processor of a monitoring device presented herein, is adapted to cause the monitoring device to measure the parameter of the medical imaging apparatus during different life cycles of the medical imaging apparatus by the sensor, and to determine, by the data processing unit, the present life cycle of the medical imaging apparatus and control the at least one sensor based on the determined present life cycle of the medical imaging apparatus.

The computer program element may be part of a computer program, but it can also be an entire program by itself. For example, the computer program element may be used to update an already existing computer program to get to the present invention.

The program element may be stored on a computer readable medium. The computer readable medium may be seen as a storage medium, such as for example, a USB stick, a CD, a DVD, a data storage device, a hard disk, or any other medium on which a program element as described above can be stored.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
Fig. 1 illustrates schematically a monitoring device according to an exemplary embodiment of the invention.
Fig. 2 illustrates schematically a method for determining a functionality of a medical imaging apparatus according to an exemplary embodiment of the invention.

### DETAILED DESCRIPTION OF EMBODIMENTS

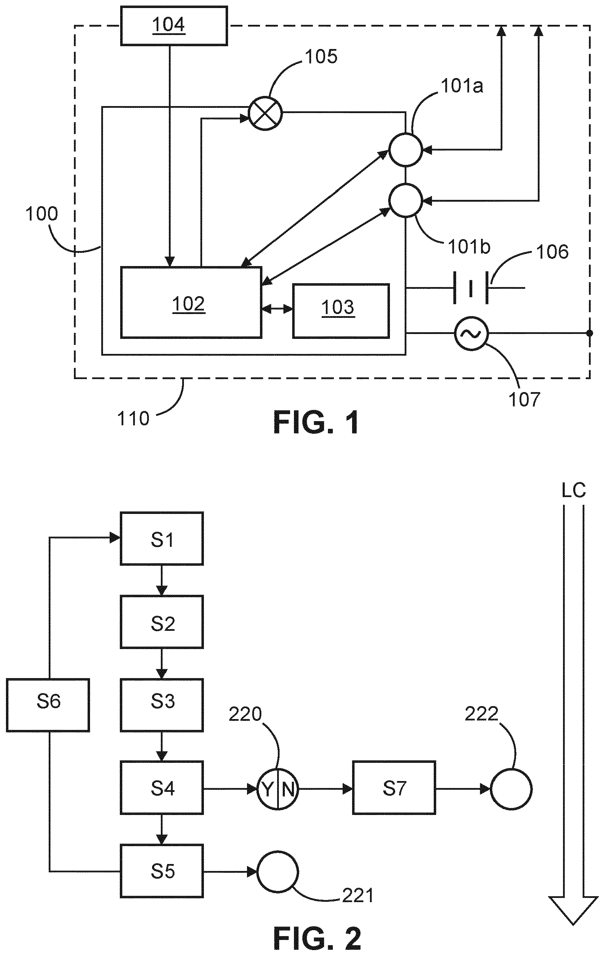

Fig. 1 illustrates a monitoring device 100 for monitoring a medical imaging apparatus 110 according to an exemplary embodiment of the present invention. The monitoring device 100 comprises at least one sensor 101 configured for measuring at least one parameter of the medical imaging apparatus 110. Further, it comprises a data processing unit 102 configured for receiving data from the at least one sensor 101 and for analyzing the received data thereby evaluating based on the analyzed data, during at least one life cycle of the medical imaging apparatus 110, whether a functionality of the medical imaging apparatus 110 is maintained. The sensor 101 is configured for measuring the parameter of the medical imaging apparatus 110 during different life cycles of the medical imaging apparatus 110. The data processing unit 102 is configured for determining the present life cycle of the medical imaging apparatus 110 and is configured for controlling the at least one sensor 101 based on the determined present life cycle of the medical imaging apparatus 110. As can be seen in Fig. 1 the illustrated monitoring apparatus comprises at least two sensors 101a and 101b for measuring a parameter of the medical imaging apparatus 110. For example, the sensors 101a, 101b may be used for measuring the same and/or different parameters of the medical imaging apparatus 110. The medical imaging apparatus 110 is illustrated as a dotted line because the monitoring device 100 may be integrated in the medical imaging apparatus 110 or not. The data processing unit may be configured for controlling the sensors 101a, 101b, an indicating unit 105 and a storage unit 105. Further, the data processing unit 102 may be configured for storing the data received from the at least one sensor 101 and/or is configured for processing the stored data for at least one of the different life cycles or preferably for all life cycles of the medical imaging apparatus 110. The monitoring device 100 may comprise the storage unit 103 configured for storing the data received from the sensor 101, wherein the storage unit 103 is illustrated in Fig. 1 as a part of the monitoring device. Contrary, the storage unit 103 may be part of the data processing unit 102, or may be an extra external part, or may be a cloud storage via an interface connection. The indicating unit 105 may be configured for indicating an optical and/or acoustical signal when the measured parameter of the medical imaging apparatus 110 differ from a predetermined signal signature, wherein the signal is indicated to user operating the monitoring device and/or the medical imaging apparatus. The indicating unit 105 may also be a signal to system control unit and the medical imaging apparatus (system) may indicate the signal and/or may be a remote signal via wired, and/or may be a wireless interface to external signal indication unit. Additionally, the data processing unit 102 may receive from an external trigger unit 104 respective signals, which may indicate the start or the end of a life cycle. The monitoring device 100 in Fig. 1 comprises a self-sustainable power supply 106 and may additionally be connected to the power supply 107 of the medical imaging apparatus, wherein the power supplies 106, 107 may interchangeable with each other.

Fig. 2 shows a flow diagram illustrating a method for evaluating functionality of a medical imaging apparatus 110 according to an exemplary embodiment of the present invention. The method comprises the step S1 of measuring at least one parameter of the medical imaging apparatus 110 by at least one sensor 101 of a monitoring device 100. The step S2 of receiving data from the at least one sensor 101 by a data processing unit 102 of the monitoring device 100. In step S3 the received data is analyzed by the data processing unit 102 of the monitoring device 100 and thereby in step S4 evaluating based on the analyzed data, during at least one life cycle LC of the medical imaging apparatus 110, whether a functionality of the medical imaging apparatus 110 is maintained. After the evaluation step S4 an output 220 may be generated indicating whether the functionality is maintained or not (indicated in Fig. 2 by "Y" yes, functionality is maintained, and "N" no, functionality may not be maintained). The output 220 may also be any kind of acoustical or optical indicator for indicating the result of the evaluation, for instance a display. Further, the measuring step S1 of the parameter of the medical imaging apparatus 110 by the sensor 101 may be conducted during different life cycles LC of the medical imaging apparatus 110. After step S4 the method further comprises step S5 of determining the present life cycle LC of the medical imaging apparatus by the data processing unit 102. The step S5 may comprise generating an output 221 indicating the respective determined life cycle LC. Afterwards the method comprises step S6, controlling the at least one sensor 101, by the data processing unit 102, based on the determined present life cycle LC of the medical imaging apparatus 110. Additionally, the method may comprise step S7 evaluating whether an existing functionality of the medical imaging apparatus 110 is maintained and conforming whether medical imaging apparatus 110 is continuously capable for applying the functionality, wherein an output 222 may be generated depending on the evaluation result of step S7. The method steps described hereinabove may be carried out during at least one life cycle LC and they may be carried out for different life cycles LC. The generated outputs 220-222 may be displayed by the indicating unit to a user or via a display of any kind of computer display device.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 100: monitoring device
- 101a, b: sensor
- 102: data processing unit
- 103: storage unit
- 104: trigger unit
- 105: indicating unit
- 106: self-sustainable power supply
- 107: power supply
- 110: medical imaging apparatus
- 220-222: output

## Claims

1. A monitoring device (100) for monitoring a medical imaging apparatus (110), the monitoring device comprising
at least one sensor (101) configured for measuring at least one parameter of the medical imaging apparatus;
a data processing unit (102) configured for receiving data from the at least one sensor (101) and for analyzing the received data thereby evaluating based on the analyzed data, during at least one life cycle of the medical imaging apparatus, whether a functionality of the medical imaging apparatus is maintained;
wherein the sensor (101) is configured for measuring the parameter of the medical imaging apparatus during different life cycles of the medical imaging apparatus;
wherein the data processing unit (102) is configured for determining the present life cycle of the medical imaging apparatus and is configured for controlling the at least one sensor based on the determined present life cycle of the medical imaging apparatus.

2. The monitoring device according to claim 1,
wherein the evaluating of the functionality of the medical imaging apparatus comprises, evaluating whether an existing functionality of the medical imaging apparatus is maintained and conforming whether medical imaging apparatus is continuously capable for applying the functionality.

3. The monitoring device according to claim 1 or 2,
wherein the different life cycles of the medical imaging apparatus are at least one of a phase of mounting the medical imaging apparatus, a phase before transporting the medical imaging apparatus, a phase of transporting and storing the medical imaging apparatus, a phase of operating the medical imaging apparatus, and a phase of maintenance of the medical imaging apparatus,
wherein for each phases the data processing unit (102) is configured for controlling the at least one sensor based on the parameter to be measured in the respective life cycle.

4. The monitoring device according to any one of the preceding claims,
wherein the measured parameter is at least one of a vibration of a component of the medical imaging apparatus, a vibration of the medical imaging apparatus, an acceleration of a component of the medical imaging apparatus, an acceleration of the medical imaging apparatus, a surrounding temperature of a component of the medical imaging apparatus, a surrounding temperature of the medical imaging apparatus, a surrounding humidity of a component of the medical imaging apparatus, a surrounding humidity of the medical imaging apparatus, a temperature of the medical imaging apparatus, a temperature of a component of the medical imaging apparatus, a mechanical shock of the medical imaging apparatus, a mechanical shock of a component of the medical imaging apparatus, a vacuum parameter of the medical imaging apparatus, an emitter parameter of the medical imaging apparatus, a power supply parameter of the medical imaging apparatus, an over/under voltage parameter of a component of the medical imaging apparatus, an over/under voltage parameter of the medical imaging apparatus, an X-ray radiation parameter of the medical imaging apparatus, a rotation speed of a component of the medical imaging of the apparatus relative to the medical imaging apparatus, a rotation speed of the medical imaging apparatus, an electron beam parameter of the medical imaging apparatus, and a magnetic field parameter of the medical imaging apparatus.

5. The monitoring device according to any one of the preceding claims,
wherein the different life cycles of the medical imaging apparatus are determined depending on the measured parameter of the at least one sensor (101) and/or determined autonomously by the monitoring device and/or are determined based on an external trigger (104),
wherein the external trigger comprises at least one of a manual activation, and an external sensor signal received by the data processing unit (102).

6. The monitoring device according to any one of the preceding claims,
wherein the data processing unit (102) is configured for controlling a signal condition of the sensor (101) and/or an analysis of the measured parameter based on the determined life cycle of the medical imaging apparatus.

7. The monitoring device according to any one of the preceding claims,
wherein the at least one sensor (101) is configured for measuring a plurality of parameters of the medical imaging apparatus, wherein the at least one of the plurality of parameters is preferably at least one of a vibration of a component of the medical imaging apparatus, a vibration of the medical imaging apparatus, an acceleration of a component of the medical imaging apparatus, an acceleration of the medical imaging apparatus, a surrounding temperature of a component of the medical imaging apparatus, a surrounding temperature of the medical imaging apparatus, a surrounding humidity of a component of the medical imaging apparatus, a surrounding humidity of the medical imaging apparatus, a temperature of the medical imaging apparatus, a temperature of a component of the medical imaging apparatus, a mechanical shock of the medical imaging apparatus, a mechanical shock of a component of the medical imaging apparatus, a vacuum parameter of the medical imaging apparatus, an emitter parameter of the medical imaging apparatus, a power supply parameter of the medical imaging apparatus, an over/under voltage parameter of a component of the medical imaging apparatus, an over/under voltage parameter of the medical imaging apparatus, an over/under current parameter of a component of the medical imaging apparatus, an over/under current parameter of the medical imaging apparatus, an X-ray radiation parameter of the medical imaging apparatus, a rotation speed of a component of the medical imaging of the apparatus relative to the medical imaging apparatus, a rotation speed of the medical imaging apparatus, an electron beam parameter of the medical imaging apparatus, and a magnetic field parameter of the medical imaging apparatus.

8. The monitoring device according to any one of the preceding claims, further comprising
a plurality of sensors (101a, 101b) configured for measuring at least one parameter and/or a plurality of parameters of the medical imaging apparatus,
wherein the monitoring device is configured for evaluating a plurality of functionalities of the medical imaging apparatus during the different life cycles of the medical imaging apparatus.

9. The monitoring device according to any one of the preceding claims,
wherein the monitoring device comprises a self-sustainable power supply (106) and/or
wherein the monitoring device is configured for being connected to a power supply (107) of the medical imaging apparatus.

10. The monitoring device according to any one of the preceding claims, further comprising
an indicating unit (105) configured for indicating an optical and/or acoustical signal when the measured parameter of the medical imaging apparatus differ from a predetermined signal signature,
wherein the predetermined signal signature depends on the phase of the life cycle.

11. A component for a medical imaging apparatus, the component comprising a monitoring device (100) according to any of the claims 1 to 10,
wherein the monitoring device is arranged at the component, and/or wherein the monitoring device is removably arranged at the component,
wherein the component is preferably an X-ray tube.

12. A medical imaging apparatus (110) comprising
a medical imaging unit for generating a medical imaging of a patient,
a monitoring device (100) according to any of the claims 1 to 10,
wherein the medical imaging apparatus is preferably a medical X-ray imaging apparatus and/or
wherein the monitoring device is arranged at the medical imaging apparatus, and/or wherein the monitoring device is removably arranged at the medical imaging apparatus.

13. A method for evaluating functionality of a medical imaging apparatus (110), the method comprising the steps of
measuring (S1) at least one parameter of the medical imaging apparatus by at least one sensor of a monitoring device,
receiving (S2) data from the at least one sensor by a data processing unit of the monitoring device,
analyzing (S3) the received data by the data processing unit of the monitoring device and thereby evaluating (S4) based on the analyzed data, during at least one life cycle of the medical imaging apparatus, whether a functionality of the medical imaging apparatus is maintained,
measuring the parameter of the medical imaging apparatus by the sensor during different life cycles of the medical imaging apparatus,
determining (S5) the present life cycle of the medical imaging apparatus by the data processing unit,
controlling (S6) the at least one sensor, by the data processing unit, based on the determined present life cycle of the medical imaging apparatus.

14. The method according to claim 13, further comprising the steps of
evaluating whether an existing functionality of the medical imaging apparatus is maintained and conforming (S7) whether medical imaging apparatus is continuously capable for applying the functionality and/or
controlling, by the data processing unit, a data acquisition rate of the sensor and/or an analysis, by the data processing unit, of the measured parameter based on the determined life cycle of the medical imaging apparatus and/or
measuring a plurality of parameters of the medical imaging apparatus by the at least one sensor and/or
measuring at least one parameter and/or a plurality of parameters of the medical imaging apparatus by a plurality of sensors and/or
evaluating a plurality of functionalities of the medical imaging apparatus during the different life cycles of the medical imaging apparatus,
wherein the different life cycles of the medical imaging apparatus are at least one of a phase of mounting the medical imaging apparatus, a phase before transporting the medical imaging apparatus, a phase of transporting and storing the medical imaging apparatus, a phase of operating the medical imaging apparatus, and a phase of maintenance of the medical imaging apparatus, and the method further comprises
controlling for each phases the at least one sensor based on the parameter to be measured in the respective life cycle by the data processing unit of the monitoring device

15. A computer program element for evaluating a functionality of a medical imaging apparatus,
wherein the computer program element, when being executed by a processor of a monitoring device, is adapted to cause the monitoring device to
measure at least one parameter of the medical imaging apparatus by at least one sensor,
receive data from the at least one sensor and for analyzing the received data, by a data processing unit, thereby evaluating based on the analyzed data, during at least one life cycle of the medical imaging apparatus, whether a functionality of the medical imaging apparatus is maintained;
measure the parameter of the medical imaging apparatus during different life cycles of the medical imaging apparatus by the sensor;
determine, by the data processing unit, the present life cycle of the medical imaging apparatus and control the at least one sensor based on the determined present life cycle of the medical imaging apparatus.
